# EUROPEAN PATENT SPECIFICATION

(11) **EP 1 405 862 B1**
(45) Date of publication and mention of the grant of the patent: **09.04.2008**
(21) Application number: 03021521.4
(22) Date of filing: 24.09.2003
(51) Int. Cl.: C07K 14/47, G01N 33/68, A61K 38/17, A61K 39/00

(54) **Method for the identification of antigenic peptides**
Verfahren zur Identifizierung antigener Peptide
Procédé d'identification des peptides antigéniques

(30) Priority: 02.10.2002 EP 02022223
(43) Date of publication of application: 07.04.2004
(60) Divisional application: 06015737.7
(73) Proprietor: F.HOFFMANN-LA ROCHE AG, 4070 Basel (CH)
(72) Inventor: Kropshofer, Harald, 79539 Loerrach (DE); Vogt, Anne, 79539 Loerrach (DE)

(56) References cited:
- WO-A-02/40647
- US-B1- 6 455 048
- KROPSHOFER H ET AL.: "Self-Release of CLIP in Peptide Loading of HLA-DR Molecules" SCIENCE, vol. 270, no. 5240, 1995, pages 1357-1359, XP008026441

## Description

The present invention relates to methods useful for isolating antigenic peptides from a limited quantity of cells or bodily fluid from a mammalian organism in an amount sufficient to determine their sequence and identity. Therefore this invention relates to methods for identifying novel disease-associated antigens, e.g. tumor antigens and antigens involved in autoimmune diseases, to be utilized for diagnostic or therapeutic purposes.

Pathological conditions, such as infectious diseases, autoimmune disorders or cancer, can be distinguished from healthy conditions by the expression of disease-specific molecules. In particular, proteins which are newly expressed, mutated or aberrantly expressed, can be utilized as markers for the respective malignancy.

A potent class of markers serving as both diagnostic and therapeutic tools are protein fragments or peptides bound to molecules of the major histocompatibility complex (MHC). In humans, MHC molecules are termed human leukocyte antigens (HLA). HLA-associated peptides are short, encompassing 9-25 amino acids (Kropshofer, H. & Vogt, A.B., Immunol Today 18 (1997) 77-82). On the one hand, these peptides are derived from self-proteins in order to establish self-tolerance. On the other hand, HLA-associated peptides are derived from foreign proteins of viral, fungal or bacterial origin in order to fight foreign invaders. Through activation of specialized immune cells, named T lymphocytes (short: T cells), HLA-peptide complexes are indispensable for mounting a cellular or humoral immune response. Particular self-peptides, denoted autoantigenic peptides, are erroneously recognized by autoaggressive T cells giving rise to autoimmune diseases. Conversely, the lack ofT cell recognition of self-peptides derived from tumor-specific antigens, contributes to immune evasion and progressive growth of tumors (Boon, T. et al., Ann Rev Immunol. 12 (1994) 337-265). Hence, increasing our SH/07.08.2003 knowledge about disease-associated marker peptides would be of considerable importance in tumor immunology and autoimmunity.

With regard to their function, two classes of MHC-peptide complexes can be distinguished (Germain, R., Cell 76 (1994) 287-299): (i) MHC class I-peptide complexes can be expressed by almost all nucleated cells in order to attract CD8+ cytotoxic T cells which lyse infected cells or tumor cells, (ii) MHC class 11-peptide complexes are constitutively expressed only on so-called antigen presenting cells (APCs), such as B lymphocytes, macrophages or dendritic cells (DCs). In particular, DCs have the capacity to prime CD4+ T helper cells (Banchereau, J. & Steinman, R.M., Nature 392 (1998) 245-254). Moreover, DCs can be licensed to optimally activate cytotoxic CD8+ T cells: this is accomplished through prior interaction of their MHC class II-peptide complexes with CD4+ T helper cells (Ridge, T. et al., Nature 393 (1998) 474-478). Thus, peptides presented by MHC class II molecules on DCs play a superior role in the pathogenesis of diseases involving T cell-driven immune responses.

The apparent role of DCs in initiating immune responses has stimulated attempts to exploit DCs as vaccines, in particular against cancer (Dallal, R.M. & Lotze, M.T., Curr Opinion Immunol 12 (2000) 583-588). A key advance was the invention of techniques for differentiation of DCs in vitro from different sources including peripheral blood, e.g. adherent monocytes, or bone marrow-derived CD34+ stem-cell precursors. DCs differentiated and activated in vitro can be used for vaccination of cancer patients after co-culture with tumor cell-derived antigens or by employing analogous techniques. Pilot dendritic cell vaccination studies have successfully induced specific anticancer responses including clinical responses (Timmermann, J.M. & Levy, R., Ann Rev Medicine 50 (1999) 507-529; Nestle, F.O., et al., Nature Medicine 7 (2001) 761-765).

DC-based cancer cell vaccines comprise DCs pulsed with normal or gene-modified cancer cells, cancer cell lysates, cancer cells fused to DCs or cancer cell-derived heat shock protein- (Hsp-) peptide complexes. The rationale behind the latter technique is that Hsps derived from tumor cells carry tumor-specific peptides which are efficiently transferred onto MHC molecules of DCs. These DCs finally prime cytotoxic T cells with anti-tumor reactivity which leads to the eradication of tumors in mice (Srivastava, P.K., et al., PNAS 83 (1986) 3407-3411; Suto, R., et al., Science 269 (1995) 1585-1588; Binder, R.J. et al, Nature Immunol. 1 (2000) 151-162).

The advantage of all these approaches is that no knowledge about the identity of tumor antigens is necessary. The disadvantage is that the identity of tumor markers remains unknown and the copy number of individual HLA-bound tumor peptides is often too low to induce long-lasting anti-tumor T cell responses.

Vaccines based on the identification of cancer antigens include DCs primed with naked DNA, recombinant adeno- or vaccinia viruses, natural or recombinant proteins purified from the respective tumor cells or synthetic analogs of tumor peptides. The advantage of pulsing DCs with antigenic tumor peptides rather than with genetic or protein precursors is that peptides can be loaded directly onto MHC molecules of DCs without further processing.

During the past decade, numerous peptides derived from tumor marker proteins and restricted by MHC class I molecules have been identified. They are grouped into four categories: cancer-testes antigens, melanoma-melanocyte differentiation antigens, mutated antigens and non-mutated shared antigens over-expressed on tumors. In several clinical pilot vaccination studies, DCs from melanoma patients were pulsed with cocktails of melanoma peptides which, as yet, were exclusively HLA class I-restricted (Nestle, F.O. et al., Nature Medicine 4 (1998) 328-332; Thurner, B. et al., J Exp Med 190 (1999) 1669-1678). However, there is increasing evidence that the efficacy and longevity of cytotoxic T cell responses against tumors can be increased by the involvement of MHC class II -restricted helper T cells. Hence, an improved vaccination method would foresee the combinatorial use of MHC class II associated tumor peptides in addition to MHC class I antigens.

Knowledge of MHC class II-restricted cancer antigens recognized by CD4+ T helper cells lags behind the identification of class I-restricted antigens (Wang, R.-F., Trends in Immunol 22 (2001) 269-276). One reason is that transfection of cDNA libraries from tumor cells into target cells and then using anti-tumor T cells to identify the appropriate transfectants and antigenic epitopes - a method successfully employed with MHC class I molecules - is not effective because the encoded proteins do not travel to the MHC class II pathway in APCs.

An innovative alternative is to use autologous DCs pulsed with tumor cells or particular tumor marker proteins and sequence the peptides associated to MHC or Hsp molecules on DCs. This approach, however, has not been employed so far, since DCs are non-dividing in vitro and only available in very small amounts from peripheral blood or bone marrow. Moreover, peptide purification and sequencing techniques were by far too insensitive, as yet, to directly identify disease-associated peptides by this or any other approach.

The same limitations are evident in the context of autoimmune diseases, such as rheumatoid arthritis (RA). RA patients suffer from systemic destruction of their joint tissue, which is mediated by auto-aggressive T lymphocytes and auto-antibodies. The presence of both auto-reactive T cells and antibodies rely on the presentation of MHC class II-restricted peptide antigen. In accordance with that, HLA-DR molecules, particularly the genes DRB1*0401 and DRB1*0404 in people of European descent, revealed to be major risk factors and confer increased susceptibility to RA (Marrack P et al. Nat. Med., 2002, 7: 899-905). Several candidate auto-antigens, such as collagen type II, fiaggrin, IgG and cartilage glycoprotein gp39, have been proposed. The corresponding autoantigenic peptides have been elucidated by indirect means only, e.g. the capacity to activate T cell clones present in serum or synovial fluid in RA patients. Recently, it became clear that none of the major CD4+ T cell clones accumulating in inflamed synovia of joints of RA patients recognizes the respective epitopes (Kotzin BL et al., PNAS (2000), 97, 291-296). A likely rationale for this is that it was not possible, as yet, to sequence HLA-DR-associated peptides from synovial tissue in RA, so that direct identification of autoantigenic peptides is still missing.

Kropshofer H et al., Science 270 (1995) 1357-1359, describe a method for isolating antigenic peptides comprising the steps of providing complexes of Hsp molecules with antigenic peptides isolated from a mammalian organism in an amount of 5 µg and sequestering the complex by immunoaffinity chromatography.

Hence, similar as in the tumor field, establishing a methodology that allows sequencing of naturally processed Hsp-associated peptides in the femtomolar range is a major need.

The present invention provides methods for isolating and identifying femtomolar amounts of peptide antigens presented by 0.1 to 5 µg Hsp receptors isolated from an organism or from cells derived from an organism. Said methods concern immune monitoring of diseases, e.g. autoimmune diseases, the design of individualized peptide vaccines for the treatment of diseases, e.g. of cancer and the quality control of vaccines e.g. those based on dendritic cells. The methods of the invention have the advantage that the identity of bound and/or presented antigenic peptides can be elucidated from very small amounts of bodily fluids or cells isolated from an mammalian organism.

The described methods ensure that the antigenic peptides isolated and identified are those that are bound and/or presented by peptide receptors in vivo or are those that are naturally-processed and presented by APCs, preferably DCs in vitro.

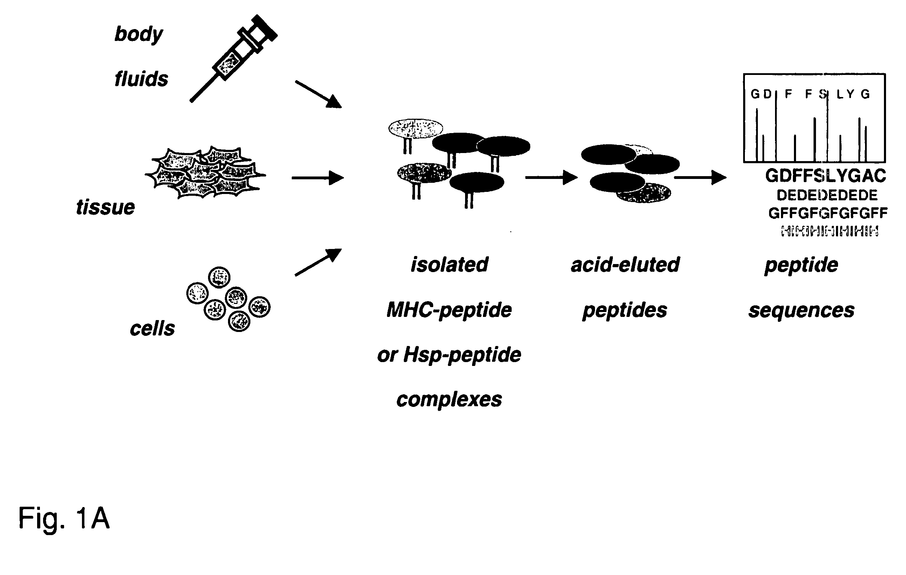

Fig. 1A is a diagram showing an overview of the methodology following strategy 1 (direct approach): Hsp-peptide complexes are isolated directly from tissue or bodily fluids thereby leading to the identification of naturally processed Hsp associated antigens presented in vivo.

### Method

The present invention provides a method for isolating disease-associated antigenic peptides in femtomolar amounts allowing their identification which method comprises providing complexes of peptide receptors with antigenic peptides from a mammalian organism in an amount of 0.1 to 5 µg, preferably in an amount of 0.2 to 3 µg. This quantity equals to the amount of material which is normally available from biopsies or bodily fluids of patients or healthy donors. The lowest amount of material necessary in the prior art is about 200 µg MHC class 11 molecules derived from an unlimited source (inbred mice) (Dongre AR et al., EJI 2001, 31, 1485-94). This is about two orders of magnitude more material than available from human patient material.

The amount of tissue or bodily fluid necessary to obtain e.g. 100 ng MHC class II molecules depends on the number of cells that do express MHC class II and on the expression rate of MHC class II molecules: e.g. 100 ng of MHC class II are equivalent to about 2 x 10⁵ mature DCs or 5 to 10 x 10⁶ peripheral blood monocytes or about 5 x 10⁷ peripheral blood mononuclear cells which can be obtained from about 50 ml of blood.

The high sensitivity required for identifying Hsp-associated peptides is explained by the fact that each type of these peptide receptors, e.g. human MHC class II gene product HLA-DR1, carries about 500 to 1000 different antigenic peptides (Chicz RM et al., J Exp. Med. 1993, 178, 27-47; Chicz RM & Urban RG, Immunol. Today, 1993, 15: 155-160). However, most of the 500 to 1000 different peptides attain very low copy numbers and, therefore, are not very likely to play a physiological role. Especially in the MHC class II field, those peptides that are of immunological relevance e.g. those that activate helper T cells, attain moderate to high copy numbers (Latek RR & Unanue ER, Immunol. Rev. 1999, 172: 209-228). These peptides cover about 40 to 50% of the total amount of peptide material eluted from MHC class II molecules and equal to about 10 to 200 individual peptides.

Many MHC class II associated peptides are represented as a set of 2 to 5 C- and N-terminal truncation variants (Rudensky AY et al, Nature 1992, 359, 429-431; Chicz et al. Nature 1992, 358: 764-768) sharing a common core sequence of about 10 to 13 amino acids which is essential for recognition by the T cell receptor. These truncation / elongation variants constitute the same T cell epitope. This means that the number of different epitopes, which are of importance is actually smaller, ranging from about 5 to 70 different epitopes. Thus, the abundance of immunologically relevant epitopes ranges from 0.2% to 5%.

In more detail, the method of the present invention comprises (a) providing complexes of Hsp 70 molecules or Hsp 90 molecules with antigenic peptides isolated from a mammalian organism in an amount of 0.1 to 5 µg, (b)- sequestering the Hsp 70 complexes by 1) ammonium sulfate precipitation, 2) application to ADP-affnitiy beads and 3) application to DEAE anion exchange beads, and Hsp 90 complexes by 1) ammonium sulfate precipitation, 2) concanavalin A affinity chromatography, and 3) DEAE anion exchange chromatography, and (c) washing the bounded complexes of Hsp molecules with antigenic peptides with water or a Tris, phosphate or acetate buffer in a concentration range of 0.5 - 10mM and eluting the associated antigenic peptides from the peptide receptors.

### Origin of the peptides

The antigenic peptides of the present invention are peptides which are associated with peptide receptors from tissue or body fluids or cells of a mammalian organism or from antigen presenting cells derived from a mammalian organism. Peptides may be bound to intracellular peptide receptors relating to the heat shock protein (Hsp) family.

The antigenic peptides comprise self antigens, tumor antigens, autoantigens, viral, bacterial, and parasitic antigens. Some antigenic peptides may induce tolerance. Other antigenic peptides may elicit an immune response and are therefore immunogenic peptides. The antigenic peptides of the present invention may be naturally processed antigenic peptides that means they are generated from antigenic proteins by the proteolytic system of the respective cell and loaded onto peptide receptors. The antigenic peptides may also be non-naturally processed synthetic or recombinant antigenic peptides which may have been administered to an organism where they have been loaded onto peptide receptors without further processing or they may have been contacted with cells expressing peptide receptors in cell culture or with isolated peptide receptors in vitro.

Therefore, the methods of the present invention comprise antigenic peptides, which are naturally-processed antigenic peptides, as well as antigenic peptides which are synthetic or recombinant antigenic peptides.

As all these peptide receptors are able to accommodate a broad variety of peptide ligands (see above), each single peptide whose sequence has to be determined is represented in only femtomolar amounts. 1 µg MHC class II (16 pmol) may carry dominant peptide species, with each single peptide attaining an occupancy of 0.1 - 2%, which equals to about 16 - 320 femtomoles. The methods of the present invention allow the isolation of these femtomolar amounts of antigenic peptides from 0.1 to 5 µg of peptide receptors loaded with peptides and their subsequent sequencing.

### Origin of the peptide receptors

In a further embodiment, the methods of the present invention relate to Hsp proteins.

Furthermore, complexes of peptide receptors with antigenic peptides may be isolated from diverse body fluids of an organism, e.g. of blood, serum, ascites, synovial fluid, from tissue samples of an organism, e.g. tumor biopsies, or from cells isolated from an organism.

Complexes of Hsp proteins with antigenic peptides may be isolated from a mammalian organism, preferably from a human organism.

The Hsp proteins isolated from an organism in an amount of 0.1 to 5 µg. Preferably, the Hsp proteins are isolated from an organism in an amount of 0.2 to 3 µg.

### Origin of the cellular material

The methods of the present invention encompass all cells expressing Hsp proteins, e.g. all cells comprising Hsp molecules with associated antigens, cells expressing Hsp molecules comprise nearly all nucleated cells.

### Solubilization of peptide receptors from cells

For the purification of peptide receptor-peptide complexes from cells or tissue, the membranes of the cells or tissue have to be solubilized. Cell lysis may be carried out with methods known in the art, e.g. freeze-and-thaw cycles and the use of detergents, and combinations thereof. Preferred lysis methods are solubilization using detergents, preferably TX-100, NP40, n-octylglucoside, Zwittergent, Lubrol, CHAPS, most preferably TX-100 or Zwittergent 3-12. Cell debris and nuclei have to be removed from cell lysates containing the solubilized receptor-peptide complexes by centrifugation. Therefore, in a further embodiment of the present invention, the complexes of peptide receptors with antigenic peptides are isolated from the cells with methods comprising solubilization with a detergent.

### Purification of Hsp-peptide complexes

Hsp-peptide complexes may be purified by methods known in the art (Binder, R. et al. J. Immunol., (2000), 165: 2582-2587). In brief, cells may be homogenized in a hypotonic buffer and fractionated by ammonium sulfate precipitation. The 50% precipitates may be applied to ADP-affinity beads and, subsequently, to DEAE anion exchange beads in ordered to purify Hsp70-peptide complexes. The 80% precipitates of the above ammonium sulfate precipitation may be used to purify Hsp90 family protein-peptide complexes by a combination of Concanavalin A affinity chromatography and DEAE anion exchange chromatography.

### Elution and fractionation of peptide receptor-associated peptides

By eluting the peptides from the Hsp molecules, a complex mixture of naturally processed peptides derived from the source of potential antigen and from polypeptides of intra or extracellular origin, is obtained. Only after elution, peptides can be fractionated and subjected to sequence analysis.

The antigenic peptides in the methods of the present invention may be eluted by a variety of methods known in the art, preferably by using diluted acid, e.g., diluted acetonitrile (Jardetzky TS et al., Nature 1991 353, 326-329), diluted acetic acid and heating (Rudensky AY et al., Nature 1991, 353, 622-626; Chicz RM et al., Nature 1992, 358, 764-768) or diluted trifluoro acetic acid at 37°C (Kropshofer H et al., J Exp Med 1992, 175, 1799-1803). Most preferably, the peptides are eluted at 37°C with diluted trifluoro acetic acid.

The sequestered Hsp receptor-peptide complexes are washed with water or low salt buffer before elution in order to remove residual detergent contaminants. The low salt buffer is a Tris, phosphate or acetate buffer in a concentration range of 0.5 - 10 mM, preferably in a concentration of 0.5 mM. In a preferred embodiment, the peptide receptor-peptide complexes are washed with ultrapure water (sequencing grade) conventionally used for HPLC analysis, preferably with ultrapure (sequencing grade) water from MERCK. The washing step may be carried out by ultrafiltration. The ultrafiltration may be carried out in an ultrafiltration tube with a cut-off of 30 kD, 20 kD, 10 kD or 5 kD, preferably of 30 kD and a tube volume of 0.5 - 1.0 ml ("Ultrafree" tubes; Millipore). The washing in the ultrafiltration tube may be carried out 4 to 12 times, preferably 6 to 10 times, with a volume of 10 to 20 times the volume of the beads carrying the receptor-peptide complexes, preferably with a volume of 15 times the beads. The eluted peptides may be separated from the remaining peptide receptor molecules using the same ultrafiltration tube. The eluted peptides may then be lyophilized.

### Peptide sequence analysis by liquid chromatography-mass spectrometry (LC-MS)

In a further embodiment of the present invention, the isolated antigenic peptides are fractionated, sequenced and identified. By sequencing it is understood that the amino acid sequence of the individual peptides in the mixture of isolated antigenic peptides is elucidated by methods adequate to sequence femtomolar amounts of peptides. By identifying it is understood that it is established from which proteins or polypeptides the antigenic peptides are derived and which sequence they constitute within these proteins or polypeptides.

In a first step, the complex mixture of eluted peptides may be fractionated by one of a variety of possible chromatographic methods, e.g. by reversed phase, anion exchange, cation exchange chromatography or a combination thereof. Preferably, the separation is performed by C18-reverse phase chromatography or by reversed-phase / cation exchange two-dimensional HPLC, denoted as MudPit (Washburn MP et al., Nat Biotechnol., (2001), 19, 242-247).

The fractionation is done in a HPLC mode utilizing fused-silica micro-capillary columns which are either connected to a nano-flow electrospray source of a mass spectrometer or to a rnicro-fractionation device which spots the fractions onto a plate for MALDI analysis.

A variety of mass spectrometric techniques are suitable, preferably MALDI-post source decay (PSD) MS or electrospray ionization tandem mass spectrometry (ESI-MS), most preferably ion-trap ESI-MS.

The sequences of the individual peptides can be determined by means known in the art. Preferably, sequence analysis is performed by fragmentation of the peptides and computer-assisted interpretation of the fragment spectra using algorithms, e.g. MASCOT or SEQUEST. Both computer algorithms use protein and nucleotide sequence databases to perform cross-correlation analyses of experimental and theoretically generated tandem mass spectra. This allows automated high through-put sequence analysis.

### Qualitative peptide analysis by MALDI mass spectrometry

For qualitative analysis of the whole peptide repertoire obtained upon elution, matrix-assisted laser desorption and ionization time-of-flight (MALDI-TOF) mass spectrometry may be carried out. Using settings that do not fragment the peptides, MALDI-TOF analysis provides a rough overview with regard to the complexity of the peptide mixture and the presence of dominant peptides.

### Quantitative peptide analysis

To estimate the quantity of single peptides eluted from peptide receptors, the run through of the micro-capillary column may be analyzed by a flow-through UV detector operated at a detection wave-length of 214 nm. For quantitation the peak areas of peptides to be analyzed are compared with peak areas of graded amounts of synthetic standard peptides.

### Strategy 1 (ex-vivo approach)

Strategy 1 of the present invention is used to isolate antigenic peptides which were loaded onto peptide receptors inside an organism (ex vivo approach, Fig. 1A).

The present invention relates to a method for isolating and identifying Hsp associated peptides in femtomolar amounts which method comprises providing 0.1 to 5 µg Hsp-peptide complexes from a mammalian organism. This amount of peptide receptors equals to the amount of material which is normally available from biopsies or bodily fluids of patients or healthy donors.

The Hsp-peptide complexes may be purified from isolated cells e.g. blood monocytes, from a mixture of cells e.g. peripheral blood mononuclear cells, from tissue, e.g. tumor biopsies, or from body fluids e.g. ascites or synovial fluid.

The body fluids may contain Hsp-peptide complexes bound to cells present in the body fluid, e.g. in synovial fluid, bound to vesicles present in the body fluid e.g. apoptotic vesicles or exosomes derived from cells (Denzer K et al., J Cell Science, 2000, 113, 3365-3374).

Hsp-peptide complexes may be isolated from diverse body fluids of an organism, e.g. of blood, serum, ascites, synovial fluid or from tissue samples of an organism, e.g. biopsies, excised primary or secondary tumors or from cells isolated from an organisms.

Complexes of peptide receptors with antigenic peptides may be isolated from cells, tissue or body fluids from a mammalian organism, preferably from a human organism.

### Applications

The methods of the present invention can be applied to identify peptides involved in the pathogenesis of a wide range of diseases.

Candidate diseases include, without limitation, autoimmune diseases (e.g. rheumatoid arthritis (RA), type I diabetes, multiple sclerosis (MS), coeliac disease, myasthenia gravis (MG) and systemic lupus erythematosus (SLE)), cancer (e.g. melanoma, breast cancer, B cell lymphomas, prostate cancer, renal cancer) or infectious diseases (e.g. diseases caused by HIV, hepatitis C virus, measles virus, mycobacteria).

One aspect is a therapeutic purpose, wherein one or more of the identified peptides are used to vaccinate patients against cancer or infectious diseases.

Alternatively, the relevant peptides may be utilized for the generation of vaccines based on DCs. In this case, autologous DCs derived from patients' monocytes may be pulsed with the relevant peptides or recombinant proteins containing the relevant peptide sequences. Nucleic acid molecules which encode the relevant peptides may be incorporated into a vector in order to transfect tumor cells. These transfected tumor cells may be fused with DCs.

A further therapeutic application of the peptides relates to the situation where identified peptides are autoantigens in the context of autoimmune diseases. In this case, complexes of the respective autoantigenic peptide and its restricting MHC class II molecules may be targeted by chimeric or humanized antibodies. This approach may lead to diminishment of autoantigenic MHC class II peptide complexes and, thus, to a decline in the number of autoaggressive CD4+ T helper cells which belong to one of the driving forces in autoimmunity.

Therefore, the present invention provides the use of the described methods for the design of individualized peptide vaccines for the treatment of diseases, preferably cancer.

Beyond that, the method of the invention can be exploited for several diagnostic purposes. First, peptides of the invention may be used as response markers to track the efficacy of a therapeutic regime. Essentially, one can determine the baseline value for the relevant peptide, e.g. an autoantigenic peptide in the synovial fluid of rheumatoid arthritis patients, using strategy 1 of the invention, administer a given therapeutic drug and then monitor levels of the autoantigenic peptide thereafter, observing changes in peptide levels as indicia of the efficacy of the regime.

Therefore, the present invention provides the use of the described methods for the control of the efficacy of a therapeutic treatment.

Autoantigenic peptides which are only found in certain stages or phases of an autoimmune disease may be utilized as stage-specific markers.

Therefore, the present invention provides the use of the described methods for immune monitoring of diseases, preferably of autoimmune diseases.

Finally, the method of the invention not only allows the identification ofMHC or Hsp-associated peptides, but, at the same time, the protein wherefrom the peptide is derived. These proteins may be used as diagnostic markers in the very same manner as described above for the corresponding peptides.

A method of producing a pharmaceutical composition may comprise the steps of the methods of the present invention, producing the identified peptides and optionally modifying them and formulating the product obtained with a pharmaceutically acceptable carrier or diluent.

Depending on the intended use of the composition, adequate carriers or diluents have to be added. Examples of such carriers and methods of formulation for pharmaceutical compositions may be found in Remington's Pharmaceutical Sciences. To form a pharmaceutically acceptable composition suitable for effective administration, such compositions will contain an effective amount of the identified substance.

While it is possible for the antigenic peptide to be administered in a pure or substantially pure form, it is preferable to present it as a pharmaceutical composition, formulation or preparation.

The pharmaceutical compositions, both for veterinary and for human use, comprise an antigenic peptide as described above, together with one or more pharmaceutically acceptable carriers and, optionally, other therapeutic ingredients. The carrier(s) must be "acceptable" in the sense of being compatible with the other ingredients of the formulation and not deleterious to the recipient thereof. The pharmaceutical compositions may conveniently be presented in unit dosage form and may be prepared by any method well-known in the pharmaceutical art.

All methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product into the desired formulation.

Formulations suitable for intravenous, intramuscular, subcutaneous, or intraperitoneal administration conveniently comprise sterile aqueous solutions of the active ingredient with solutions which are preferably isotonic with the blood of the recipient. Such formulations may be conveniently prepared by dissolving solid active ingredient in water containing physiologically compatible substances such as sodium chloride (e.g. 0.1-2.0M), glycine, and the like, and having a buffered pH compatible with physiological conditions to produce an aqueous solution, and rendering said solution sterile. These may be present in unit or multi-dose containers, for example, sealed ampoules or vials.

The formulations of the present invention may incorporate a stabilizer. Illustrative stabilizers are polyethylene glycol, proteins, saccharides, amino acids, inorganic acids, and organic acids which may be used either on their own or as admixtures. These stabilizers are preferably incorporated in an amount of about 0.11 to about 10,000 parts by weight per part by weight of immunogen. If two or more stabilizers are to be used, their total amount is preferably within the range specified above. These stabilizers are used in aqueous solutions at the appropriate concentration and pH. The specific osmotic pressure of such aqueous solutions is generally in the range of about 0.1 to about 3.0 osmoles, preferably in the range of about 0.8 to about 1.2. The pH of the aqueous solution is adjusted to be within the range of about 5.0 to about 9.0, preferably within the range of 6-8. In formulating the antigenic peptide of the present invention, anti-adsorption agent may be used.

## Claims

1. A method for isolating antigenic peptides in femtomolar amounts, which method comprises
(a) providing complexes of Hsp 70 molecules or Hsp 90 molecules with antigenic peptides isolated from a mammalian organism by solubulization of cells or tissue in an amount of 0.1 to 5 µg and
(b) sequestering the Hsp 70 complexes by 1) ammonium sulfate precipitation, 2) application to ADP-affnitiy beads and 3) application to DEAE anion exchange beads, and Hsp 90 complexes by 1) ammonium sulfate precipitation, 2) concanavalin A affinity chromatography, and 3) DEAE anion exchange chromatography,
(c) washing the bounded complexes of Hsp molecules with antigenic peptides with water or a Tris, phosphate or acetate buffer in a concentration range of 0.5 - 10mM and eluting the associated antigenic peptides from the Hsp molecules.

2. The method according to claim 1, wherein the bounded complexes of Hsp molecule with antigenic peptides are washed in an ultrafiltration tube 4 to 12 times with a volume 10 to 20 times the volume of the beads used for sequestering the receptor-peptide complexes

3. The method according to claim 1, wherein the bounded complexes of Hsp molecule with antigenic peptides are washed in an ultrafiltration tube 6 to 10 times with a volume 10 to 20 times the volume of the beads used for sequestering the receptor-peptide complexes.

4. The method according to claim 1 to 3, wherein the water of (c) is ultrapure water.

5. The method according to any one of claims 1 to 4, which method comprises isolating the complexes of Hsp molecules with antigenic peptides in an amount of 0.1 to 5 µg from cells isolated from a mammalian organism.

6. The method according to claim 5, wherein the cells isolated from a mammalian organism are dendritic cells.

7. The method according to any one of claims 1 to 6, wherein the antigenic peptides are eluted from the Hsp molecules using diluted acid.

8. The method according to any one of claims 1 to 7, wherein the isolated antigenic peptides are fractionated, sequenced and identified.

9. The method according to claim 8, wherein the isolated antigenic peptides are fractionated, sequenced and identified by methods comprising liquid chromatography and mass spectrometry.

10. The method according to any one of claims 1 to 9, wherein the antigenic peptides are naturally-processed antigenic peptides or non-naturally processed antigenic peptides administered to the organism.

11. The method according to claims 1 to 10, wherein the mammalian organism is a human organism.

12. Use of the methods of claims 1 to 11 for immune monitoring of diseases.

13. Use of the methods of claims 1 to 11 for the control of the efficacy of a therapeutic treatment.

14. Use of the methods of claims 1 to 11 for the design of individualized peptide vaccines for the treatment of diseases.

## Patentansprüche

1. Verfahren zum Isolieren von antigenen Peptiden in femtomolaren Mengen, wobei das Verfahren
(a) das Bereitstellen von Komplexen von Hsp-70-Molekülen oder Hsp-90-Molekülen mit antigenen Peptiden, isoliert aus einem Säugerorganismus, durch Solubilisierung von Zellen oder Gewebe in einer Menge von 0,1 bis 5 µg, und
(b) das Sequestrieren der Hsp-70-Komplexe durch 1) Ammoniumsulfatausfällung, 2) Aufbringung auf ADP-Affinitäts-Kügelchen und 3) Aufbringung auf DEAE-Anionenaustauschkügelchen, und der Hsp-90-Komplexe durch 1) Ammoniumsulfatausfällung, 2) Concanavalin-A-Affinitätschromatographie und 3) DEAE-Anionenaustauschchromatographie,
(c) das Waschen der gebundenen Komplexe von Hsp-Molekülen mit antigenen Peptiden mit Wasser oder einem Tris-, Phosphat- oder Acetatpuffer in einem Konzentrationsbereich von 0,5 bis 10 mM und das Eluieren der assoziierten antigenen Peptide aus den Hsp-Molekülen umfaßt.

2. Verfahren nach Anspruch 1, wobei die gebundenen Komplexe des Hsp-Moleküls mit antigenen Peptiden in einem Ultrafiltrationsröhrchen vier- bis zwölfmal mit einem 10- bis 20fachen Volumen des Volumens der Kügelchen, die zum Maskieren der Rezeptor-Peptid-Komplexe verwendet werden, gewaschen werden.

3. Verfahren nach Anspruch 1, wobei die gebundenen Komplexe des Hsp-Moleküls mit antigenen Peptiden in einem Ultrafiltrationsröhrchen sechs- bis zehnmal mit einem 10- bis 20fachen Volumen des Volumens der Kügelchen, die zum Maskieren der Rezeptor-Peptid-Komplexe verwendet werden, gewaschen werden.

4. Verfahren nach den Ansprüchen 1 bis 3, wobei das Wasser von (c) ultrareines Wasser ist.

5. Verfahren nach einem der Ansprüche 1 bis 4, wobei das Verfahren das Isolieren der Komplexe von Hsp-Molekülen mit antigenen Peptiden in einer Menge von 0,1 bis 5 µg aus Zellen, die aus einem Säugerorganismus isoliert wurden, umfaßt.

6. Verfahren nach Anspruch 5, wobei die Zellen, die aus einem Säugerorganismus isoliert wurden, dendritische Zellen sind.

7. Verfahren nach einem der Ansprüche 1 bis 6, wobei die antigenen Peptide aus den Hsp-Molekülen unter Verwendung verdünnter Säure eluiert werden.

8. Verfahren nach einem der Ansprüche 1 bis 7, wobei die isolierten antigenen Peptide fraktioniert, sequenziert und identifiziert werden.

9. Verfahren nach Anspruch 8, wobei die isolierten antigenen Peptide durch Verfahren, die Flüssigchromatographie und Massenspektrometrie umfassen, fraktioniert, sequenziert und identifiziert werden.

10. Verfahren nach einem der Ansprüche 1 bis 9, wobei die antigenen Peptide natürlich prozessierte antigene Peptide oder nicht-natürlich prozessierte antigene Peptide, die an den Organismus verabreicht werden, sind.

11. Verfahren nach den Ansprüchen 1 bis 10, wobei der Säugerorganismus ein menschlicher Organismus ist.

12. Verwendung der Verfahren nach den Ansprüchen 1 bis 11 zur Immunüberwachung von Krankheiten.

13. Verwendung der Verfahren nach den Ansprüchen 1 bis 11 zur Kontrolle der Wirksamkeit einer therapeutischen Behandlung.

14. Verwendung der Verfahren nach den Ansprüchen 1 bis 11 zur Konstruktion individualisierter Peptidimpfstoffe zur Behandlung von Krankheiten.

## Revendications

1. Procédé d'isolement de peptides antigéniques en des quantités femtomolaires, ce procédé comprenant
(a) la fourniture de complexes de molécules de Hsp 70 ou de molécules de Hsp 90 avec des peptides antigéniques isolés à partir d'un organisme de mammifère par solubilisation de cellules ou de tissu en une quantité de 0,1 à 5 µg et
(b) la séquestration des complexes de Hsp 70 par 1) précipitation avec du sulfate d'ammonium, 2) application à des billes d'affinité à l'ADP et 3) application à des billes d'échange d'anions sur DEAE, et des complexes de Hsp 90 par 1) précipitation avec du sulfate d'ammonium, 2) chromatographie d'affinité à la concanavaline A et 3) chromatographie par échange d'anions sur DEAE,
(c) le lavage des complexes liés de molécules de Hsp avec des peptides antigéniques avec de l'eau ou un tampon Tris, phosphate ou acétate dans un domaine de concentration de 0,5 - 10 mM et élution des peptides antigéniques associés à partir des molécules de Hsp.

2. Procédé selon la revendication 1, dans lequel on lave 4 à 12 fois les complexes liés de molécules de Hsp avec des peptides antigéniques dans un tube d'ultrafiltration avec un volume de 10 à 20 fois le volume des billes utilisées pour la séquestration des complexes récepteur-peptide.

3. Procédé selon la revendication 1, dans lequel on lave 6 à 10 fois les complexes liés de molécules de Hsp avec des peptides antigéniques dans un tube d'ultrafiltration avec un volume de 10 à 20 fois le volume des billes utilisées pour la séquestration des complexes récepteur-peptide.

4. Procédé selon les revendications 1 à 3, dans lequel l'eau de (c) est de l'eau ultrapure.

5. Procédé selon l'une quelconque des revendications 1 à 4, ce procédé comprenant l'isolement des complexes de molécules de Hsp avec des peptides antigéniques en une quantité de 0,1 à 5 µg à partir de cellules isolées à partir d'un organisme de mammifère.

6. Procédé selon la revendication 5, dans lequel les cellules isolées à partir d'un organisme de mammifère sont des cellules dendritiques.

7. Procédé selon l'une quelconque des revendications 1 à 6, dans lequel on élue les peptides antigéniques à partir des molécules de Hsp à l'aide d'un acide dilué.

8. Procédé selon l'une quelconque des revendications 1 à 7, dans lequel on fractionne, on séquence et on identifie les peptides antigéniques isolés.

9. Procédé selon la revendication 8, dans lequel on fractionne, on séquence et on identifie les peptides antigéniques isolés par des procédés comprenant la chromatographie liquide et la spectrométrie de masse.

10. Procédé selon l'une quelconque des revendications 1 à 9, dans lequel les peptides antigéniques sont des peptides antigéniques maturés de manière naturelle ou des peptides antigéniques maturés de manière non naturelle administrés à l'organisme.

11. Procédé selon les revendications 1 à 10, dans lequel l'organisme de mammifère est un organisme humain.

12. Utilisation des procédés des revendications 1 à 11 pour le suivi immun de maladies.

13. Utilisation des procédés des revendications 1 à 11 pour le contrôle de l'efficacité d'un traitement thérapeutique.

14. Utilisation des procédés des revendications 1 à 11 pour la conception de vaccins peptidiques individualisés destinés au traitement de maladies.
